# EUROPEAN PATENT SPECIFICATION

(11) **EP 1 414 356 B1**
(45) Date of publication and mention of the grant of the patent: **19.10.2005**
(21) Application number: 02749099.4
(22) Date of filing: 31.07.2002
(51) Int. Cl.: A61B 17/17, A61B 17/15

(54) **GUIDE FOR LOCATING FEMUR RESECTION PLANE**
FÜHRUNG ZUR LOKALISIERUNG DER RESEKTIONSFLÄCHEN DES FEMUR
GUIDE DE LOCALISATION DE PLAN DE RESECTION DE FEMUR

(30) Priority: 10.08.2001 GB 0119541
(43) Date of publication of application: 06.05.2004
(73) Proprietor: Depuy International Limited, Beeston, Leeds LS11 8DT (GB)
(72) Inventor: AUGER, Daniel, Fort Wayne, IN 46814 (US); HELDRETH, Mark, Mentone, IN 46539 (US)
(74) Representative: Belcher, Simon James
(86) International application number: PCT/GB2002/003515
(87) International publication number: WO 2003/013373

(56) References cited:
- EP-A- 0 327 387
- EP-A- 0 709 061
- EP-A- 0 914 806
- WO-A-98/32384
- FR-A- 2 705 885

## Description

This invention relates to a guide for use in determining the location of a plane for resecting the femur in a knee joint, relative to a reference surface on the tibia, to prepare the femur for implantation of the femoral component of a knee joint prosthesis.

Accurate resection of a patient's bones is crucially important for success in an operation to implant a joint prosthesis. The resection plane must be accurately located: it is generally desirable to minimise the amount of bone that is removed, while being sure to remove all bone tissue that is defective. The orientation of the plane relative to the anatomical axis must also be accurately controlled to ensure alignment of the articulating surfaces of the joint after surgery, throughout the full range of joint motion.

Resection of the femur for implantation of a knee joint prosthesis is performed on at least two planes, arranged adjacent to one another around the or each condyle. Generally, the location of the resection plane for the tibia is determined first, and the location of the femoral resection plane is then determined with reference to the tibial resection plane, generally after resection of the tibia. Accurate location of the tibial resection plane in a knee joint is commonly established using an alignment guide which includes an alignment rod which can be fastened to the tibia distally, close to the ankle. The rod extends along the tibial, parallel to the axis of the tibia. The resection plane can then be defined relative to the tibial axis using a cutting block which can be attached to the alignment guide.

The location of the femoral resection plane relative to the tibial resection plane will depend on factors which include the dimensions of the implant components that are to be used. Appropriate alignment and spacing of the femoral and tibial components should be maintained throughout the range of motion of the knee joint. This requires accurate location of anterior and posterior femoral resection planes on the femoral component. In addition to the dimensions of the implant component, this can be affected by features of the patient's anatomy. For example, the alignment of the femoral resection planes can be arranged to provide a correction for varus and valgus deformations. It can also be required to ensure that the implanted femoral component is aligned with the natural condyle, to facilitate optimum coverage of femoral tissue and that the extent of overhang of the component beyond the femoral tissue is minimised.

The femoral resection is performed using a cutting block which provides a surface to define the resection plane. The cutting block is attached to the femur before the resection is performed, for example using fixation pins which are inserted through fixation holes in the block. The position and orientation of the resection plane is controlled relative to the tibia by referring to the tibia when the cutting block is mounted on the femur. EP-A-0327387 (The preamble of claim 1 is based on this document.) discloses a securement technique for securing a prosthetic femoral component wherein the knee joint is exposed and a plane facet cut on the tibial plateau. This facet serves as a basis of securement of a tibial component for association with the subject femoral component. A jig comprising an alignment part and a drill guide part is applied to locate a shoe part between the plane facet and the posterior portion of the femoral condyle wherein said jig extends parallel to the longitudinal axis of the femoral shaft. Fixation pins extending perpendicularly from the longitudinal axis of the jig are then inserted into the femur through fixation holes in the guide part of the jig. This method therefore positions and orientates the resection plane relative to the tibia.

The present invention provides a guide which can be used to locate the cutting block on the femur, which includes a limb which can be inserted between the femur and the tibia, in contact with the tibia, in which the surface of the limb which contacts the tibia has at least two generally flat portions so that the surface is generally convex.

Accordingly the invention provides a guide for use in determining the location of a plane for resecting one of the femoral condyles in a knee joint, relative to a reference surface on the tibia, to prepare the condyle for implantation of the femoral components of a unicompartmental knee joint prosthesis, in which the reference surface is provided by the part of the area of the tibia which faces towards one of the femoral condyles where the tibia has been resected to receive the tibial component of the knee joint prosthesis, the remainder of the area of the tibia being provided by the tibial bearing surface which remains intact for bearing engagement with the corresponding femoral condyle, the guide comprising:
a. a first limb which is in contact with the reference surface on the tibia, and
b. a second limb extending generally perpendicularly from the first limb so as to extend over the femur when the first limb is inserted between the femur and the tibia, which has at least two holes formed in it to locate holes on the femur, characterised in that said first limb can be inserted between the femur and the tibia. and said first limb has a femoral surface which faces towards the femur and a tibial surface which faces towards the tibia,
in which the first limb is configured to fit between the reference surface of the tibia and the respective condyle, and in which the tibial surface of the first limb has at least two generally flat portions when the limb is viewed in cross-section looking along its length so that the tibial surface is generally convex, the included angle defined by the flat portions being at least about 165°.

The guide of the invention has the advantage that it enables a surgeon to move the guide between positions in which different flat portions are in contact with the reference surface of the tibia, which correspond to different orientations of the cutting block to provide different resection planes. The different orientations can facilitate optimum coverage of femoral tissue. It can also help to minimise the extent of overhang of the component beyond the femoral tissue, and can be used to correct deformations. The provision of the flat portions enables the surgeon to feel when the guide is in different orientations which correspond to respective positions of the cutting block. For example, one of the flat portions can correspond to a position of the cutting block in which the resection plane is perpendicular to the anatomical axis, and another of the flat portions can correspond to a position of the cutting block in which the angle between the resection plane and the anatomical axis is, for example 15°.

The guide of the present invention is able to adopt different orientations relative to the anatomical features of the patient's knee by virtue of the fact that it is configured for use specifically in a unicompartmental knee joint replacement. In contrast with guide blocks which are used for total knee joint replacement, the guide of the present invention has a single limb for fitting between the tibia and the femur, and the guide can be moved between its different orientations while that limb is in contact with the reference surface. This is not possible with existing guide blocks in which the limb (or limbs) which fit between the tibial and femoral surfaces extends across both of the condyles.

The flat portions need not be absolutely flat. For example, when the first limb is viewed in cross-section, the flat portions might be slightly rounded. For many applications, this will not be preferred. When the flat portions are intended to fit against a flat resected surface on the tibia, it is preferred that the portions are as close as possible to flat so that the engagement with that surface is more positive.

The ability of the guide of the present invention to provide different orientations for a cutting block finds particular application in the implantation of a knee prosthesis in just one of the joint compartments, leaving the other condyle and the corresponding part of the tibia intact. It is important then to ensure that the alignment of the bearing surfaces in the undisturbed compartment is not affected adversely by misalignment of the bearing surfaces on the prosthesis components.

The guide of the invention is configured so that it fits between the reference surface of the tibia and the respective condyle. The reference surface is provided by a part of the tibia from which the bearing surface has been resected, in order subsequently to receive the tibial component of the unicompartmental knee joint prosthesis. The remainder of the tibial bearing surface is left substantially (more generally, entirely) intact for bearing engagement with the corresponding femoral condyle. The width of the first limb will be depend on the size of the patient. Generally, the width of the first limb will be not more than about 35 mm, preferably not more than about 30 mm, especially not more than about 25 mm. The width of the first limb will generally be at least about 10 mm, preferably at least about 15 mm. The width of the first limb can vary along the length of the limb; for example, the limb might be tapered along its length. The dimensions referred to above for the width of the limb will then be average widths. When calculating the average width of the limb, localised variations (for example localised bulges or indentations) are disregarded.

Each of the width of each of the flat portions (measured in the plane of the respective flat portion) on the first limb will be not more than about 15 mm, preferably not more than about 10 mm, especially not more than about 8 mm.

The first limb will generally be provided by a unitary structure. However, the limb might be slit along its length. Generally, any such slit will be narrow so that the entire first limb can fit between the reference surface on the tibia and one of the condyles.

Preferably, the plane defined by one of the flat portions is generally perpendicular to the axis defined by the second limb. The axis of the second limb can then be aligned with the anatomical axis, and this can locate the cutting block so that the resection plane is perpendicular to the anatomical axis. An arrangement in which the second limb is aligned with the anatomical axis to locate the resection plane perpendicular to the axis has the advantage of ease of use for the surgeon.

Preferably, the tibial surface has three generally flat portions when the first limb is viewed in cross-section looking along its length so that the tibial surface is generally convex, the included angle defined by each pair of adjacent flat portions being at least about 165°. Preferably, the included angles defined the pairs of adjacent flat portions are the same.

The included angle between a pair of adjacent flat portions can be 165° which can provide a difference of 15° between orientations of the cutting block (and therefore between the resection planes) in which the flat portions of the tibial surface are in contact with the reference surface on the tibia. A guide with a larger included angle can be used, for example 170°, to provide a smaller difference, for example 10°, between orientations of the cutting block.

The guide can be used to prepare the femur for subsequent fixation to it of a cutting block. For example, the guide can have holes formed in it which can be used to locate fixation holes in the femur for the cutting block. This can be achieved by drilling fixation holes into the femur through the holes in the guide block. The holes can receive fixation pins (which might be threaded in the form of screws), by which the cutting block can be fastened to the femur. The guide can also be used to locate a hole for a lug on the femoral component of the knee joint prosthesis.

Accordingly, in another aspect, the invention provides a guide of the kind discussed above and a cutting block which can be fastened to the femur and which has at least one slot formed in it for guiding a saw blade during resection of the femur, the cutting block including locating pins which can extend into fixation holes at locations on the femur defined using the guide. The locating pins can be provided separate from the cutting block, or they can be provided integrally with it so that they are fastened to the block.

Alternatively, the second limb of the guide itself can have at least one slot in it for guiding a saw blade during resection of the femur so that the guide itself can function as the cutting block. In this case, holes in the guide can be used to fasten it to the femur, for example by means of pins (which might be threaded in the form of screws) inserted through the said holes.

The guide of the invention finds particular application when preparing the femur for the posterior resection. The guide can be used to locate the plane for the posterior resection while the knee is flexed, allowing the first limb of the guide to be placed between the posterior portion of the femoral bearing surface and the tibial reference surface, and the second limb of the guide to extend towards the anterior portion of the femoral bearing surface. After the posterior cut has been made, additional cuts can be made to enable a femoral prosthesis component to be fitted. These additional cuts are often referred to as chamfer cuts: a posterior chamfer cut can be located posteriorly of the posterior cut which faces the tibia when the knee is flexed through about 90°, and an anterior chamfer cut can be located between the posterior and anterior cuts. A cutting block to assist in locating the chamfer cuts can be combined with the cutting block which is used to locate the posterior cut, by the provision of multiple guide slots.. Alternatively, one or more additional cutting blocks can be used. The guide that is used to locate the fixation points for the posterior cut cutting block can also be used to locate the fixation points for the or each chamfer cutting block.

The guide will generally be made from a metal, such as is commonly used in the manufacture of surgical instruments.

Embodiments of the present invention will now be described by way of example with reference to the accompanying drawings, in which:
Figure 1 is an isometric view of the guide of the present invention.
Figure 2 is a schematic cross-sectional view of a tibia and femur viewed along the M-L axis.
Figure 3 is a view of a patient's knee, in flexion, with the guide of the invention positioned between the femur and the tibia.

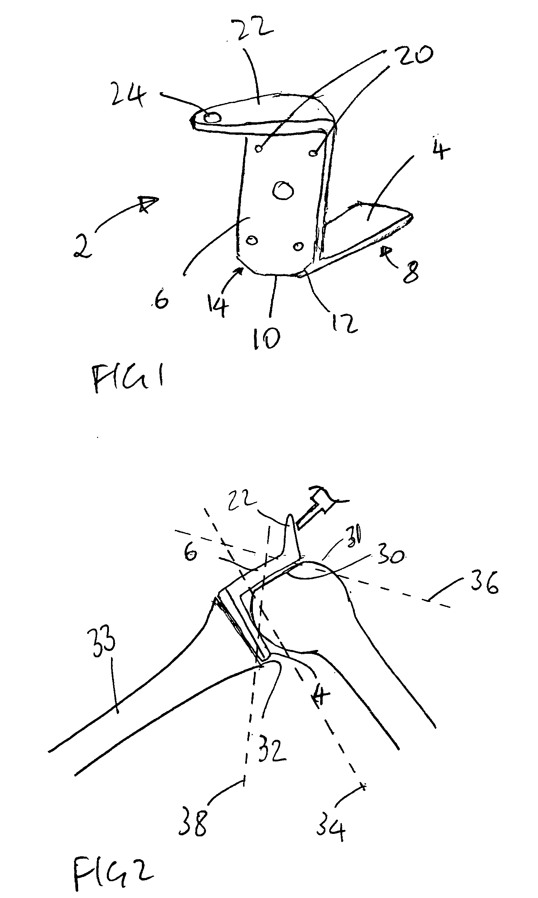

Referring to the drawings, Figure 1 shows a guide 2 which comprises a first limb 4 and a second limb 6. The limbs are arranged approximately perpendicularly to one another.

The tibial surface 8 of the first limb 4 has three flat portions when it is viewed in cross-section looking along its length. The central portion 10 is generally parallel to the opposite surface of the first limb. The edge portions 12, 14 which extend along opposite edges of the first limb (with the central portion between them) are each arranged so that the included angle between each edge portion and the central portion is 170°. The tibial surface therefore has a generally convex shape when the first limb is viewed in cross-section.

The second limb 6 has fixation holes 20 formed in it through which a drill or other marker can pass to make holes in a bone which the second limb is forced against when in use.

In the embodiment shown in Figure 1, the guide includes a third limb 22 by which the guide can be gripped for manipulation during surgery. The third limb has an aperture 24 in it for engaging a manipulation instrument.

The guide that is shown in Figure 1 can be used to position a cutting guide on a femur, to locate the appropriate resection plane for the posterior portion of the condyle. This part of a knee joint replacement procedure is commonly performed after resection of the tibia and after resection of the anterior portion of the condyle. Location of the tibial section plane can be performed using a resection guide such as that disclosed in the patent application filed with the present application entitled "Tibial resection guide", which bears the case reference P11654. Subject matter disclosed in the specification of that application is incorporated in the specification of the present application by this reference.

Referring to Figure 2, the anterior cut 30 of the femoral condyle 31 is performed relative to the plane 32 on which the tibia 33 is resected, using an anterior cutting guide which can be inserted between the resected tibia and the femur, whose thickness is related to the thickness of the prosthesis components that are to be implanted. The anterior cutting guide is used to locate a cutting block on the femur, which has a slot in it for receiving a saw blade. The anterior cutting guide is used in this way with the knee joint extended.

The guide 2 of the present invention is used to position the cutting block which defines the plane 34 for the posterior cut. Subsequently, a cutting block which defines the planes 34, 38 for the anterior and posterior chamfer cuts is fixed to the femur, and the location of that cutting block can again be determined using the guide of the present invention.

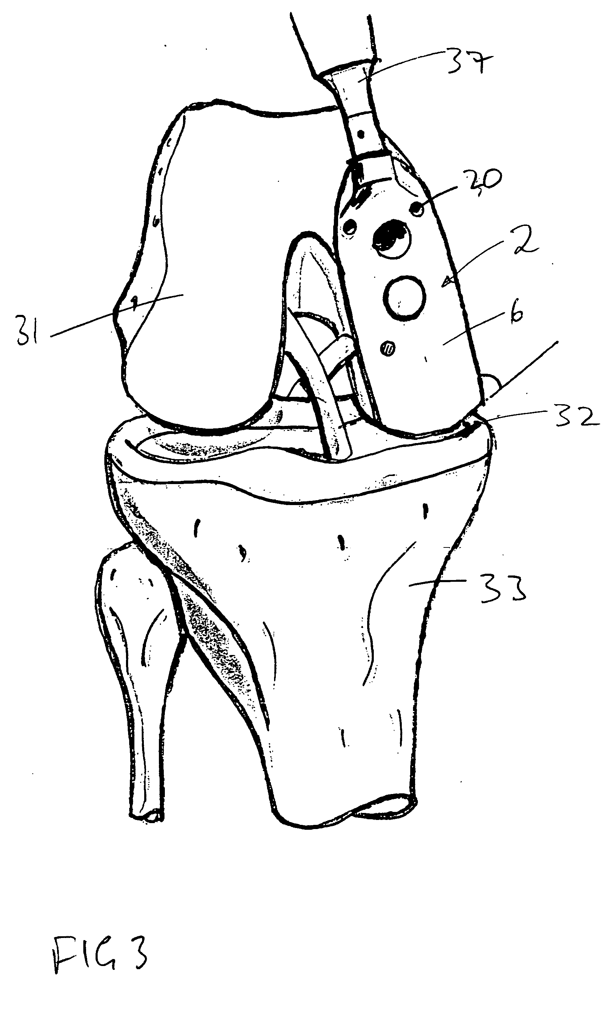

Referring to Figure 3, the guide 2 of the present invention is positioned on the resection plane 32 of the tibia while the knee joint is flexed using a positioning instrument 37. The first limb of the guide is located on the resected plane 32, with the lower surface 8 in contact with the tibia on the resection plane and the opposite surface of the first limb directed towards the femur, in the region of the posterior part of the condyle. As shown, one of the edge portions 14 of the lower surface of the first limb of the-guide is in contact with the tibia, so that the second limb extends from the tibial resection plane 32 inclined to the perpendicular. The tibial resection plane will generally extend perpendicular to the anatomical axis. If, instead, the central portion of the lower surface of the first limb is in contact with the tibia, the second limb will extend generally perpendicular to the tibial resection plan, approximately parallel to the anatomical axis.

The thickness of the first limb of the guide will be selected according to the maximum spacing that is required between the resected tibia and femur to receive the joint prosthesis components. If the surgeon determines that less space between the resected bones is required, for example because of the small size of the bones or because the condition of the bone tissue is good, so that less bone need be resected, spacers can be positioned with the first limb between the femur and the tibia. The guide of the invention can include a plurality of such spacers, for example with a range of thicknesses. Suitable spacers might have a thickness of 1 mm, 2 mm, 3 mm and 4 mm. When such spacers are positioned with the first limb between the femur and the tibia, the cutting block is located closer to the femoral bearing surface, meaning that less bone is resected.

The surgeon can use the guide of the invention to select a desired orientation of the femoral component of a knee joint prosthesis, for example depending on the geometry of the condyle in the patient's joint. This can help to maintain optimum geometry of both bearing surfaces throughout the articulation of the joint. The orientation of the femoral component can be selected by moving the guide so that, instead of the central portion 10 of the first surface 8 being in contact with the tibia, one of the edge portions 12, 14 is in contact. This will correspond to movement of the cutting block (and therefore of the posterior resection plane) through 10°. The surgeon might consider that an intermediate movement is appropriate, and can use the limits of movement (0° and 10°) to gauge a degree of movement that is appropriate for a particular patient.

When the orientation of the guide on the first surface has been established, holes are drilled in the femur through the fixation holes 20. A cutting block can subsequently be fixed to the femur using the holes. The location of the holes is such that the position of the cutting block, with its slot in it for guiding a saw blade, is appropriate to provide the desired resection plane.

A common femoral component design requires four cuts which are shown in Figure 3. These are performed in the order (1) anterior cut 30, (2) posterior cut 34, (3) posterior chamfer 36, and (4) anterior chamfer 38. The guide of the present invention can be used to position one or more cutting block which can be used to locate cuts (2) to (4). It can also locate guides for use in other steps of the procedure, such as for example the location of a guide for drilling a hole to receive a lug which penetrates into a natural femoral tissue (a "femoral lug" as is known in femoral components of existing knee joint prostheses).

## Claims

1. A guide (2) for use in determining the location of a plane for resecting one of the femoral condyles in a knee joint, relative to a reference surface on the tibia, to prepare the condyle for implantation of the femoral component of an unicompartmental knee joint prosthesis, in which the reference surface is provided by the part of the area of the tibia which faces towards one of the femoral condyles where the tibia has been resected to receive the tibial component of the knee joint prosthesis, the remainder of the area of the tibia being provided by the tibial bearing surface which remains intact for bearing engagement with the corresponding femoral condyle, the guide comprising:
a. a first limb (4) which is in contact with the reference surface on the tibia (32) and
b. a second limb (6) extending generally perpendicularly from the first limb so as to extend over the femur (31) when the first limb (4) is inserted between the femur and the tibia (33), which has at least two fixation holes (20) formed in it to locate holes on the femur (31),
**characterised in that** said first limb can be inserted between the femur (31) and the tibia (33) and said first limb has a femoral surface which faces towards the femur (31) and a tibial surface which faces towards the tibia (33) in which the first limb is configured to fit between the reference surface of the tibia and the respective condyle, and in which the tibial surface of the first limb has at least two generally flat portions when the limb is viewed in cross-section looking along its length so that the tibial surface is generally convex, the included angle defined by the flat portions being at least about 165°.

2. A guide (2) as claimed in claim 1, in which the plane defined by one of the flat portions is generally perpendicular to the axis defined by the second limb.

3. A guide (2) as claimed in claim 1, in which the tibial surface has three generally flat portions when the first limb (4) is viewed in cross-section looking along its length so that the tibial surface is generally convex, the included angle defined by each pair of the adjacent flat portions being at least about 165°.

4. A guide (2) as claimed in claim 1, which includes spacer elements which can be positioned with the first limb (4) between the tibia (33) and the femur (31) to ensure a desired spacing between the two bones to receive a joint prosthesis.

5. A guide (2) as claimed in claim 1, in which the second limb (6) thereof has at least one slot in it for guiding a saw blade during resection of the femur (31).

6. A kit which comprises a guide (2) as claimed in claim 1 and a cutting block which can be fastened to the femur (31) and which has at least one slot formed in it for guiding a saw blade during resection of the femur (31), the cutting block including locating pins which can extend into fixation holes at locations on the femur (31) defined using the guide (2).

7. A kit as claimed in claim 6, in which the locating pins are formed integrally on the cutting block.

8. A kit as claimed in claim 6, in which the locating pins are provided separate from the cutting block, and the cutting block has holes formed in it for receiving the locating pins.

9. A kit as claimed in claim 6, in which the cutting block and the guide are provided as separate parts which can be connected together.

10. A kit as claimed in claim 6, in which the cutting block and the guide are provided as a single component.

## Patentansprüche

1. Führung (2) zur Verwendung beim Bestimmen des Ortes einer Ebene zum Resezieren einer der Femurkondylen in einem Kniegelenk, relativ zu einer Referenzfläche auf dem Schienbein, um die Kondyle für die Implantation der Oberschenkelkomponente einer einkammerigen Kniegelenk-Prothese vorzubereiten, wobei die Referenzfläche durch den Teil der Fläche des Schienbeins gebildet wird, der einer der Femurkondylen zugewandt ist, wo das Schienbein reseziert worden ist, um die Schienbeinkomponente der Kniegelenk-Prothese aufzunehmen, wobei der Rest der Fläche des Schienbeins durch die Schienbein-Anlagefläche gebildet wird, die für das Anlegen der entsprechenden Femurkondyle unversehrt bleibt, wobei die Führung aufweist:
a. eine erste Extremität (4), die in Kontakt mit der Referenzfläche auf dem Schienbein (32) ist, und
b. eine zweite Extremität (6), die sich im allgemeinen senkrecht von der ersten Extremität erstreckt, so daß sie sich über den Oberschenkel (31) erstreckt, wenn die erste Extremität (4) zwischen den Oberschenkel und das Schienbein (33) eingefügt wird, wobei sie wenigstens zwei Befestigungslöcher (20) hat, die darin gebildet sind, um Löcher auf dem Oberschenkel (31) festzulegen,
**dadurch gekennzeichnet, daß** die erste Extremität zwischen dem Oberschenkel (31) und dem Schienbein (33) eingesetzt werden kann und die erste Extremität eine Oberschenkelfläche hat, die dem Oberschenkel (31) zugewandt ist und eine Schienbeinfläche, die dem Schienbein (33) zugewandt ist, wobei die erste Extremität so ausgebildet ist, daß sie zwischen die Referenzfläche des Schienbeins und der jeweiligen Kondyle paßt, und wobei die Schienbeinfläche der ersten Extremität wenigstens zwei im allgemeinen flache Bereiche hat, wenn die Extremität im Querschnitt gesehen wird, wobei entlang ihrer Länge geschaut wird, so daß die Schienbeinfläche im allgemeinen konvex ist, wobei der eingeschlossene Winkel, der durch die flachen Bereiche definiert ist, wenigstens ungefähr 165° beträgt.

2. Führung (2) nach Anspruch 1, bei der die Ebene, die durch einen der flachen Bereiche definiert ist, im allgemeinen senkrecht zu der Achse ist, die durch die zweite Extremität definiert ist.

3. Führung (2) nach Anspruch 1, bei der die Schienbeinfläche drei im allgemeinen flache Bereiche hat, wenn man die erste Extremität (4) im Querschnitt sieht, wobei entlang ihrer Länge betrachtet wird, so daß die Schienbeinfläche im allgemeinen konvex ist, wobei der eingeschlossene Winkel, der durch jedes Paar der benachbarten flachen Bereiche definiert ist, wenigstens ungefähr 165° beträgt.

4. Führung (2) nach Anspruch 1, welche Abstandhalterelemente umfaßt, die zwischen der ersten Extremität (4) zwischen dem Schienbein (33) und dem Oberschenkel (31) positioniert werden können, um einen gewünschten Abstand zwischen den beiden Knochen zu gewährleisten, damit eine Gelenk-Prothese aufgenommen wird.

5. Führung (2) nach Anspruch 1, bei der ihre zweite Extremität (6) wenigstens einen Schlitz aufweist, um ein Sägeblatt während der Resektion des Oberschenkels (31) zu führen.

6. Einbausatz, der eine Führung (2) nach Anspruch 1 und einen Schneidblock aufweist, der an dem Oberschenkel (31) befestigt werden kann und der wenigstens einen Schlitz hat, der darin ausgebildet ist, um ein Sägeblatt während der Resektion des Oberschenkels (31) zu führen, wobei der Schneidblock Positionierstifte umfaßt, die sich in Befestigungslöchern an Orten auf dem Oberschenkel (31) erstrecken können, welche durch Verwenden der Führung (2) definiert sind.

7. Einbausatz nach Anspruch 6, bei dem die Positionierstifte einstückig auf dem Schneidblock gebildet sind.

8. Einbausatz nach Anspruch 6, bei dem die Positionierstifte getrennt von dem Schneidblock vorgesehen sind und der Schneidblock in ihm ausgebildete Löcher zum Aufnehmen der Positionierstifte hat.

9. Einbausatz nach Anspruch 6, bei dem der Schneidblock und die Führung als getrennte Teile vorgesehen sind, die miteinander verbunden werden können.

10. Einbausatz nach Anspruch 6, bei dem der Schneidblock und die Führung als eine einzige Komponente vorgesehen sind.

## Revendications

1. Guide (2) pour une utilisation dans la détermination de la localisation d'un plan pour resecter un des condyles fémoraux dans une articulation de genou, par rapport à une surface de référence sur le tibia, pour préparer le condyle à une implantation du composant fémoral d'une prothèse d'articulation de genou unicompartimentale, dans lequel la surface de référence est fournie par la partie de la surface du tibia qui est tournée vers un des condyles fémoraux où le tibia a été resecté pour recevoir le composant tibial de la prothèse d'articulation de genou, le reste de la surface du tibia étant fourni par la surface portante tibiale qui reste intacte pour porter la mise en prise avec le condyle fémoral correspondant, le guide comprenant :
a. un premier membre (4) qui est en contact avec la surface de référence sur le tibia (32) et
b. un second membre (6) s'étendant généralement perpendiculairement au premier membre de manière à s'étendre sur le fémur (31) quand le premier membre (4) est inséré entre le fémur et le tibia (33), qui a au moins deux orifices de fixation (20) formés à l'intérieur pour localiser les orifices sur le fémur (31),
**caractérisé en ce que** ledit premier membre peut être inséré entre le fémur (31) et le tibia (33), et ledit premier membre a une surface fémorale qui est tournée vers le fémur (31) et une surface tibiale qui est tournée vers le tibia (33), dans lequel le premier membre est configuré pour s'ajuster entre la surface de référence du tibia et le condyle respectif, et dans lequel la surface tibiale du premier membre a au moins deux parties généralement plates quand le membre est visualisé en section transversale en regardant le long de sa longueur, de sorte que la surface tibiale est généralement convexe, l'angle inclus défini par les parties plates étant d'au moins environ 165°.

2. Guide (2) selon la revendication 1, dans lequel le plan défini par une des parties plates est généralement perpendiculaire à l'axe défini par le second membre.

3. Guide (2) selon la revendication 1, dans lequel la surface tibiale a trois parties généralement plates quand le premier membre (4) est visualisé en section transversale en regardant le long de sa longueur, de sorte que la surface tibiale est généralement convexe, l'angle inclus défini par chaque paire des parties plates adjacentes étant d'au moins environ 165°.

4. Guide (2) selon la revendication 1, qui comprend des éléments espaceurs qui peuvent être positionnés avec le premier membre (4) entre le tibia (33) et le fémur (31) pour assurer un espacement souhaité entre les deux os pour recevoir une prothèse d'articulation.

5. Guide (2) selon la revendication 1, dans lequel le second membre (6) de celui-ci a au moins une fente pour guider une lame de scie durant la resection du fémur (31).

6. Kit qui comprend un guide (2) selon la revendication 1 et un bloc de découpage qui peut être attaché au fémur (31) et qui a au moins une fente formée dans celui-ci pour guider une lame de scie durant la resection du fémur (31), le bloc de découpage comprenant des broches de localisation qui peuvent s'étendre dans les orifices de fixation aux emplacements sur le fémur (31) définis en utilisant le guide (2).

7. Kit selon la revendication 6, dans lequel les broches de localisation sont formées intégralement sur le bloc de découpage.

8. Kit selon la revendication 6, dans lequel les broches de localisation sont fournies distinctes du bloc de découpage, et le bloc de découpage a des orifices formés à l'intérieur pour recevoir les broches de localisation.

9. Kit selon la revendication 6, dans lequel le bloc de découpage et le guide sont fournis en parties distinctes qui peuvent être raccordées ensemble.

10. Kit selon la revendication 6, dans lequel le bloc de découpage et le guide sont fournis en un simple composant.
